# EUROPÄISCHE PATENTSCHRIFT

(11) **EP 1 587 969 B1**
(45) Veröffentlichungstag und Bekanntmachung des Hinweises auf die Patenterteilung: **11.10.2017**
(21) Anmeldenummer: 04704542.2
(22) Anmeldetag: 23.01.2004
(51) Int. Cl.: C25D 13/02

(54) **VERFAHREN ZUM HERSTELLEN VON METALLISCHEN FORMKÖRPERN MIT EINER KERAMISCHEN SCHICHT, METALLISCHER FORMKÖRPER UND DESSEN VERWENDUNG**
METHOD FOR PRODUCING METALLIC MOULDED BODIES COMPRISING A CERAMIC LAYER, METALLIC MOULDED BODY, AND THE USE OF THE SAME
PROCEDE DE FABRICATION DE CORPS MOULES METALLIQUES POURVUS D'UNE COUCHE CERAMIQUE, CORPS MOULE METALLIQUE ET UTILISATION DE CE DERNIER

(30) Priorität: 24.01.2003 DE 10302943; 16.09.2003 DE 10343034
(43) Veröffentlichungstag der Anmeldung: 26.10.2005
(73) Patentinhaber: eZelleron Inc., Hartford, CT 06120-1537 (US)
(72) Erfinder: CLASEN, Rolf, 66386 St. Ingbert (DE); KÜHN, Sascha, 66123 Saarbrücken (DE)
(74) Vertreter: Hübsch, Dirk
(86) Internationale Anmeldenummer: PCT/DE2004/000103
(87) Internationale Veröffentlichungsnummer: WO 2004/067808

(56) Entgegenhaltungen:
- EP-A- 0 200 242
- EP-A- 0 446 999
- DE-A- 10 044 163
- DE-A- 19 524 750
- US-B1- 6 217 732
- KUEHN S ET AL: "Impregnation of nickel foils with nanocrystalline ceria as anodes for solid oxide fuel cells SOFC" CERAM. ENG. SCI. PROC. (USA), CERAMIC ENGINEERING AND SCIENCE PROCEEDINGS, 2003, AMERICAN CERAMIC SOC, USA, Bd. 24, Nr. 3, 26. Januar 2003 (2003-01-26), Seiten 305-310, XP001194516 ISSN: 0196-6219
- CZERWINSKI F ET AL: "Optimizing properties of CeO2 sol-gel coatings for protection of metallic substrates against high temperature oxidation" THIN SOLID FILMS, ELSEVIER-SEQUOIA S.A. LAUSANNE, CH, Bd. 289, Nr. 1, 30. November 1996 (1996-11-30), Seiten 213-219, XP004055570 ISSN: 0040-6090
- PATENT ABSTRACTS OF JAPAN Bd. 2002, Nr. 03, 3. April 2002 (2002-04-03) -& JP 2001 316874 A (ARACO CORP), 16. November 2001 (2001-11-16) in der Anmeldung erwähnt

## Beschreibung

Die Erfindung betrifft ein Verfahren zum Herstellen von metallischen Formkörpern mit einer keramischen Schicht nach dem Membranverfahren, bei dem eine poröse metallische Membran zwischen Anode und Kathode angeordnet wird, um die von H⁺ und OH⁻-Ionen hervorgerufene Änderung des pH-Wertes im Bereich der Elektroden vom Ort der Abscheidung räumlich zu trennen, einen metallischen Formkörper mit einer keramischen Schicht und die Verwendung eines derartigen metallischen Formkörpers.

Die US 6,217,732 B1 beschreibt ein durch ein Elektrophoreseverfahren mit einer partikularen Beschichtung versehenen Erzeugnis.

Aus der DE 100 44 163 A1 ist ein Verfahren zur Herstellung von porösen SiO₂-Grünkörpern mit extrem hoher Gründichte oder porösen SiO₂-Grünkörpern mit einem gezielt einstellbaren Dichtegradienten innerhalb des Grünkörpers bekannt.

In Czerwinski F. et al.: "Optimizing properties of CeO2 sol-gel coatings for protection of metallic substrates against high temperature oxidation", THIN SOLID FILMS, ELSEVIER-SEQUOIA S.A., LAUSANNE, CH, Bd. 189, Nr. 1, 30. November 1996, Seiten 213-219, CP004055570 ISSN: 0040-6090 wird die Optimierung von Eigenschaften von Sol-Gel-Beschichtungen auf CeO₂-Basis für die Beschichtung metallischer Substrate beschrieben.

Die DE 195 24 750 A1 beschreibt ein Verfahren zur elektrophoretischen Beschichtung eines Trägersubstrates, wobei ein ungesintertes poröses Trägersubstrat elektrophoretisch beschichtet und nachfolgend gesintert wird.

Aus der US 5,211 922 A ist ein Verfahren zur Herstellung eines Elements zur Mikrofiltration, Ultrafiltration und Umkehrosmose bekannt, das aus einer semipermeablen anorganischen Membran auf einem leitenden porösen Träger besteht.

Die DE 195 24 750 A1 beschreibt das Erzeugen einer keramischen Schicht auf der Oberfläche eines keramischen Formkörpers, indem Pulverteilchen durch elektrophoretische Abscheidung auf eine metallische Membran aufgebracht werden. Dazu wird eine stabilisierte Suspension genutzt.

Die DE 195 20 458 A1 beschreibt eine Vorrichtung zur elektrophoretischen Beschichtung von Substraten, welche zwei Hauptelektroden zur Erzeugung eines homogenen elektrischen Feldes und Anordnungsmittel, um ein Substrat innerhalb des homogenen elektrischen Bereiches des elektrischen Feldes zu plazieren. Darüber hinaus sind Mittel zur Homogenisierung des elektrischen Feldes vorgesehen, beispielsweise Hilfselektroden, die der Verzerrung des elektrischen Feldes infolge des Substrates entgegenwirken.

Aus der US 5,002,647 A ist ein Verfahren zum Erzeugen dicker Schichten bekannt, bei dem Pulver eines Ausgangsmaterials in Lösemittel eingebracht wird und dann zwischen den in der Lösung angeordneten Elektroden ein elektrisches Potential aufgebracht wird, wodurch das Pulver auf einem mit der Kathode verbundenen Substrat abgelagert wird. Für dieses Verfahren wird ein spezielles Lösemittelsystem vorgeschlagen.

Die JP 2001316874 A beschreibt ein entsprechendes Verfahren, bei dem im Innern eines nichtleitenden porösen Substrates eine Elektrode angeordnet wird, so daß bei Anlegung eines Potentials das Substrat beschichtet wird.

Diese Druckschriften befassen sich jedoch mit der konventionellen elektrophoretischen Abscheidung, wohingegen im vorliegenden Falle die elektrophoretische Abscheidung nach dem Membranverfahren, wie es aus der EP 0 446 999 B1 bekannt ist, erfolgt.

Hierbei wird im Gegensatz zu der konventionellen elektrophoretischen Abscheidung eine Membran zwischen Anode und Kathode angeordnet, um die von H⁺ und OH⁻-Ionen hervorgerufene Änderung des pH-Wertes im Bereich der Elektroden vom Ort der Abscheidung räumlich zu trennen. Auch die Gasentwicklung, die aufgrund der elektrolytischen Zersetzung des Wassers stattfindet, kann durch diese räumliche Trennung nicht die Teilchenbewegung und Abscheidung beeinflussen.

Poröse metallische Formkörper werden aufgrund ihrer hohen Stabilität bei gleichzeitig geringer Dichte in vielen technischen Bereichen genutzt. Als Beispiele seien Filtermembranen, Konstruktionselemente in Leichtbauweise, Implantatwerkstoffe und Anoden für Festoxid-Brennstoffzellen (Solid Oxide Fuel Cells, SOFC) genannt.

In vielen Fällen ist es erforderlich oder von großem Interesse, eine zweite (beispielsweise keramische) Phase einzubringen, die katalytische Eigenschaften, Biokompatibilität oder auch andere physikalische Eigenschaften (insbesondere Ionenleitung) mit sich bringt und/oder eine bessere Anhaftung zu dem umgebenden Material ermöglicht.

Bei temperaturbelasteten Materialverbunden (z.B. Anode-Elektrolytschicht in der SOFC) ist ein gradierter Übergang zwischen den Materialien mit unterschiedlicher thermischer Ausdehnung wünschenswert, um Spannungen und Rißbildung zu vermeiden. Für die Verwendung der metallischen Formkörper als Anoden in Festoxid-Brennstoffzellen ist eine zusätzliche keramische Komponente erforderlich. Sie ermöglicht die Ionenleitung im genutzten Volumen der Anode und verbessert das Anhaften der Elektrolytschicht.

Es gibt zwei prinzipiell unterschiedliche Anodentypen: Tragende Anoden tragen das ganze 3-Schicht-System der SOFC und haben eine Dicke zwischen 300 µm und 1.500 µm. Nicht tragende Anoden, wie sie beispielsweise in der EP 0 829 103 B1 beschrieben werden, sind dünner, etwa 50 µm.

Drei verschiedene Aufbauten der Mikrostruktur sind bekannt. Entweder nutzt man eine Mischung der keramischen und der metallischen Partikel (z.B. EP 0 525 844 B1). Anoden aus einfachen Gemischen von Keramik und Metall haben jedoch zahlreiche Nachteile: Zunächst, daß man an die Perkolationstheorie gebunden ist und nur knapp die notwendige Porosität von 30 % einstellen kann. Des weiteren neigt dieser Aufbau zu Überpotentialverlusten. Auch ist in diesem Aufbau das Metall nicht vor Korrosion geschützt.

Alternativ ist es möglich, die Mikrostruktur aufzubauen, indem ein keramisches Gerüst teilweise von Metall umgeben oder mit Metall beschichtet wird bzw. indem ein metallisches Gerüst mit einer Keramik beschichtet wird. Diese beiden Alternativen sind der Mischung der keramischen und metallischen Partikel vorzuziehen, jedoch viel aufwendiger in der Herstellung. Ein bedeutender Vorteil des beschichteten metallischen Gerüstes besteht darin, daß das Metall vor korrosivem Angriff geschützt ist, der seit einiger Zeit als Grund für die Degradation bzw. das Versagen von SOFC-Schichten verantwortlich gemacht wird.

Folgende Herstellungsverfahren sind hierfür bekannt:
1. Herstellung von tragenden Anoden durch Pressen oder Heißpressen. Mikrostruktur: Gemisch.
2. Herstellung von tragenden Anoden durch thermische Spritzverfahren, insbesondere Plasmaspritzen. Mikrostruktur: Gemisch.
3. Auftragen der Anoden als Nickelschlicker auf die Elektrolytschicht. Es folgt eine sehr aufwendige und kostenintensive Imprägnierung der Anode mit YSZ durch elektrochemische Gasphasenabscheidung EVD bei hohen Temperaturen. Die so hergestellten Anoden erreichen nach heutigem Stand die höchste Effizienz. Mikrostruktur: Metallgerüst mit Keramiküberzug.
4. Anodenherstellung mittels Foliendruck. Mikrostruktur: Gemisch.
5. Aufbau von Anoden als Gemisch zweier Phasen.
6. Imprägnierung von gesinterten Nickelmembranen mit stabilisierter YSZ-Suspension durch Tränken, wie in der EP 0 439 938 B1 dargestellt.

Nachteilig sind bei dem 3. Verfahren die hohen Kosten und der beträchtliche Zeitaufwand, die entstehenden giftigen Gase und die schlechte Kontrollierbarkeit. Eine gradierte Beschichtung kann mit diesem Verfahren nicht hergestellt werden.

Das 6. Verfahren erfordert zwei Sinterschritte. Durch Tränken können nur geringe Gründichten erreicht werden.

Alle bekannten Verfahren haben den Nachteil, daß kein gradierter Dichteverlauf in den Formkörpern erzielt werden kann.

Aufgabe der Erfindung ist es somit, ein kostengünstiges, schnelles und möglichst schadstofffreies Verfahren zum Herstellen von metallischen Formkörpern nach dem Membranverfahren mit einer keramischen Schicht ausgehend von einer porösen metallischen Membran zu schaffen. Zudem sollen die Eindringtiefe, die Gründichte und die Abscheidegeschwindigkeit der keramischen Partikel in der Metallmembran steuerbar sein.

Diese Aufgabe wird erfindungsgemäß dadurch gelöst, daß die poröse metallische Membran durch eine elektrophoretische Abscheidung von keramischen Partikeln in den Poren der metallischen Membran nachverdichtet wird und daß die metallische Membran zur elektrophoretischen Abscheidung nach dem Membranverfahren (EP 200 242 A1) zwischen zwei Elektroden angeordnet wird, wobei in diesem Fall eine metallische Membran, insbesondere eine metallische Membran in Verbindung mit einer zweiten oder mehreren metallischen oder nichtmetallischen Membranen, verwendet werden kann. Der Raum zwischen einer Elektrode und der zu imprägnierenden Seite der metallischen Membran wird mit einer Dispersion gefüllt, welche die in den Poren abzuscheidenden keramischen Partikel und ein Dispergiermittel enthält. Zusätzlich können Zusatzstoffe (insbesondere Dispergierhilfsmittel) hinzugefügt werden. Die Membran liegt dabei auf einem Potential, das > 0 bis 90 % (bezogen auf die Elektrode in der Ausgleichskammer) der an den Elektroden angelegten Spannung entspricht.

Das erfindungsgemäße Verfahren führt durch Ablagerung der keramischen Partikel in den Poren der metallischen Membran zu einer Verdichtung des Formkörpers. Abhängig von den Prozeßparametern, wie z.B. elektrisches Feld, Füllgrad der Dispersion, Teilchendurchmesser, Zetapotential, etc., sowie den Eigenschaften der Membran, wie Porenradienverteilung und Gründichte variieren die imprägnierte bzw. nachverdichtete Tiefe und der Anstieg der Gründichte. Überraschenderweise gelang mit dem erfindungsgemäßen Verfahren auch die Abscheidung von keramischen Teilchen innerhalb einer Randschicht in den Poren der metallischen Membran. Dadurch gelingt es, einen metall-keramischen Stoffverbund schnell, sauber und kostengünstig herzustellen.

Durch Anlegen eines elektrischen Feldes wird die Bildung der keramischen Schicht steuerbar. Durch das elektrische Feld werden die keramischen Teilchen beschleunigt und können tiefer in die Poren der Randschicht eindringen. Somit ist es möglich, einen Formkörper der oben beschriebenen Art mit einem gezielt eingestellten Gradienten der keramischen Imprägnierung zu schaffen.

Eine Weiterbildung der Erfindung besteht darin, daß die metallische Membran vor dem Einbringen zwischen die Elektroden mit einer Lösung getränkt wird.

Im Rahmen der Erfindung ist vorgesehen, daß das Dispergiermittel ein polares oder unpolares organisches Lösungsmittel oder Wasser ist.

Es ist vorteilhaft, daß das Lösungsmittel aus der Gruppe der Alkohole, Ester, Ether, organischen Säuren, gesättigten oder ungesättigten Kohlenwasserstoffe und Wasser ausgewählt ist oder eine Mischung hiervon ist, vorzugsweise, daß das Lösungsmittel aus der Gruppe bestehend aus Methanol, Ethanol, Propanol, Azeton und Wasser ausgewählt ist oder eine Mischung hiervon ist.

Besonders bevorzugt sind Azeton und Wasser sowie deren Mischungen als Lösungsmittel, ganz besonders bevorzugt wird Wasser.

Erfindungsgemäß ist vorgesehen, daß die Viskosität der Dispersion zwischen 1 und 1.000 mPa.s liegt, vorzugsweise zwischen 1 und 100 mPa.s liegt.

Es kann auch vorteilhaft sein, daß nacheinander verschiedene Dispersionen eingesetzt werden.

Dies kann insbesondere vorgenommen werden, um anschließend eine keramische Schicht auf eine Anode aufzubringen.

Durch das erfindungsgemäße Verfarhen ergibt sich eine gute Verankerung der keramischen Schicht, welche bei Vorliegen einer bimodalen Teilchenverteilung (bei der ein weiteres Pulver mit größeren Teilchen vorliegt, um die sich die kleineren Teilchen anordnen) noch verbessert werden kann. Neben der besseren Schichthaftung ergibt sich weiterhin eine durch Füllgrad und Zusatzstoffe der Suspension, Teilchengrößenverteilung in der Suspension (insbesondere durch Einbringen sinteraktiver Pulver, z.B. nano-CeO₂, nano-GDC) und Abscheideparameter der Elektrophorese anpassbare Gründichte der zusätzlich aufgebrachten keramischen Schicht und dadurch eine anpassbare Schrumpfung beim Sintern. Dadurch wird im Gegensatz zu der DE 195 24 750 A1 das Aufbringen und Sintern in einem Schritt möglich.

Zweckmäßig ist, daß für die elektrophoretische Abscheidung elektrische Gleichspannungen von 5 V bis 100 V bzw. eine elektrische Feldstärke von 0,1 V/cm bis 20 V/cm angelegt werden.

Weiterhin ist vorgesehen, daß die Abscheidedauer zwischen einer Sekunde und 30 Minuten beträgt.

Zur Erfindung gehörig ist ebenfalls, daß die metallischen Formkörper nach der elektrophoretischen Abscheidung einer Sinterung unterzogen werden.

Bei dem erfindungsgemäßen Verfahren ergibt sich beim Sintern fast keine Durchbiegung des Probenkörpers, wodurch die Gefahr der Rißbildung deutlich verringert wird. Es kann vorteilhaft sein, die Sinterung nicht vollständig durchzuführen, um eine gewisse Restporosität der keramischen Schicht zu erhalten.

Hierbei ist es sinnvoll, daß die Sintertemperatur zwischen 900°C und 1.700°C, vorzugsweise zwischen 1.000°C und 1.400°C, beträgt.

Eine bevorzugte Ausführungsform der Erfindung besteht darin, daß der metallische Formkörper aus Nickel besteht.

Derartige Formkörper können beispielsweise durch Pressen aus Nickelpulver hergestellt werden.

Als keramische Partikel werden vorzugsweise Ceroxidpartikel (CeO₂), insbesondere Gadolinium dotierte Cerpartikel (GDC) oder Zirkonoxidpartikel, insbesondere Yttrium stabilisiertes Zirkonoxid (YSZ) eingesetzt.

Es hat sich als besonders vorteilhaft erwiesen, daß die keramischen Partikel in bimodaler Verteilung vorliegen. Dies bedeutet, daß zwei Pulver mit unterschiedlichen mittleren Teilchendurchmessern verwendet werden.

Im Rahmen der Erfindung liegt auch ein metallischer Formkörper mit einer keramischen Schicht, wobei der metallische Formkörper eine gemäß dem erfindungsgemäßen Verfahren hergestellte Nickel-GDC-Verbundmembran ist und nach der Trocknung eine graduelle Dichteänderung von 20 Vol.-% an der imprägnierten Oberfläche bis zu einer Tiefe von etwa 200 µm aufweist.

Die zusätzliche keramische Schicht kann auch in einem Schritt mit der Imprägnierung aufgebracht werden, wenn die Suspension eine bimodale Teilchenverteilung aufweist und einen hohen Anteil sehr feiner Partikel enthält.

Bei dem metallischen Formkörper kann es sich um einen Nickelkörper mit einer Ceroxidbeschichtung, vorzugsweise mit einer bimodalen Ceroxidbeschichtung handeln. Insbesondere kann es sich um eine Nickel-GDC-Verbundmembran handeln.

Gleichfalls liegt auch die Verwendung eines derartigen erfindungsgemäßen metallischen Formkörpers, insbesondere mit einer zusätzlichen keramischen Schicht, als Anode für Festoxid-Brennstoffzellen (SOFC) im Rahmen der Erfindung.

Erfindungsgemäß kann ein derartiger metallischer Formkörper mit zusätzlich aufgebrachter keramischer Schicht auch als Anode-Elektrolyt-Schichtverbund für Festoxid-Brennstoffzellen (SOFC) verwendet werden.

Zusammenfassend bestehen die Vorteile der Erfindung darin, daß im Rahmen der Erfindung ein kostengünstiges, schnelles und schadstofffreies Verfahren zur Herstellung von metallischen Formkörpern mit einer keramischen Schicht geschaffen wurde, wobei die Eindringtiefe, die Gründichte und die Abscheidegeschwindigkeit der keramischen Partikel in der Metallmembran durch die Prozeßparameter steuerbar sind.

Nachfolgend wird die Erfindung anhand von Ausführungsbeispielen näher erläutert.

### Ausführungsbeispiele

### Beispiel 1:

Eine Dispersion aus Gadolinium dotiertem Ceroxidpulver (GDC), das einen mittleren Teilchendurchmesser von 300 nm aufweist und Wasser mit einem Feststoffgehalt von 60 Gew.-% wird hergestellt und mit 0,2 Gew.-% TMAH stabilisiert. Eine Ausgleichslösung mit bidestilliertem Wasser, versetzt mit 0,2 Gew.-% TMAH, wird hergestellt. Eine Nickelmembran wird durch uniaxiales Pressen aus Nickelpulver, das einen mittleren Teilchendurchmesser von 45 µm aufweist, hergestellt.

Die Nickelmembran, die zuvor in der Ausgleichslösung getränkt wurde, wird zwischen den Elektroden eingespannt, wodurch sich der Raum zwischen den beiden Elektroden der Elektrophoresezelle in zwei Kammern im Verhältnis 2:3 unterteilt.

Die Dispersion wird in die größere Kammer der Elektrophoresezelle eingefüllt. Die andere Kammer wird mit der Ausgleichslösung befüllt. Der Abstand zwischen den beiden Elektroden beträgt insgesamt 6 cm. An den Elektroden der Elektrophoresekammer wird dann eine Gleichspannung von 20 V für die Dauer von zwei Minuten angelegt.

Die so hergestellte Nickel-GDC-Verbundmembran weist nach der Trocknung eine graduelle Dichteänderung von 20 Vol.-%, an der imprägnierten Oberfläche bis zu einer Tiefe von etwa 200 µm auf. In REM-Aufnahmen ist nach dem Sintern bei 1.250°C eine zusammenhängende Schicht von GDC mit Vermikularstruktur zu erkennnen.

### Beispiel 2:

Eine Dispersion mit bimodaler Teilchenverteilung aus GDC-Pulvern, wobei das gröbere der Pulver einen mittleren Teilchendurchmesser von 1.000 nm und das feine Pulver einen mittleren Teilchendurchmesser von 15 nm aufweist, wird in Wasser hergestellt und mit 0,2 Gew.-% TMAH stabilisiert. Dazu werden 20 Gew.-% des feinen und 20 Gew.-% des groben Pulvers in Wasser dispergiert. Eine Ausgleichslösung mit bidestilliertem Wasser, versetzt mit 0,2 Gew.-% HCl, wird hergestellt. Eine Nickelmembran wird durch uniaxiales Pressen aus Nickelpulver, das einen mittleren Teilchendurchmesser von 45 µm aufweist, hergestellt.

Die Nickelmembran, die zuvor in der Ausgleichslösung getränkt wurde, wird zwischen den Elektroden eingespannt, wodurch sich der Raum zwischen den beiden Elektroden der Elektrophoresezelle in zwei Kammern im Verhältnis 2:3 unterteilt.

Die Dispersion wird in die größere Kammer der Elektrophoresezelle eingefüllt. Die andere Kammer wird mit der Ausgleichslösung befüllt. Der Abstand zwischen den beiden Elektroden beträgt insgesamt 6 cm. An den Elektroden der Elektrophoresekammer wird dann eine Gleichspannung von 15 V für die Dauer von drei Minuten angelegt.

Zusätzlich zu der Imprägnierung konnte eine Schicht von 150 µm Dicke aufgebracht werden.

Die Probe wird im Exsikkator bei Raumtemperatur 24 h getrocknet. Durch die hohe Gründichte läßt sich die Schicht trocknen ohne zu reißen. Beim Sintern bei 1.350°C wird das Substrat nicht belastet und man erhält eine dichte Schicht auf dem Substrat, die durch den darunter befindlichen GDC-Gradienten sehr gut verankert ist und gut anhaftet.

## Patentansprüche

1. Verfahren zum Herstellen von metallischen Formkörpern mit einer keramischen Schicht nach dem Membranverfahren, bei dem eine poröse metallische Membran zwischen Anode und Kathode angeordnet wird, um die von H⁺ und OH⁻-Ionen hervorgerufene Änderung des pH-Wertes im Bereich der Elektroden vom Ort der Abscheidung räumlich zu trennen, **dadurch gekennzeichnet, daß** die poröse metallische Membran durch eine elektrophoretische Abscheidung von keramischen Partikeln in den Poren der metallischen Membran nachverdichtet wird und daß die metallische Membran zur elektrophoretischen Abscheidung zwischen zwei Elektroden angeordnet wird und der Raum zwischen einer Elektrode und der metallischen Membran mit einer Dispersion gefüllt wird, welche die in den Poren abzuscheidenden keramischen Partikel und ein Dispergiermittel enthält.

2. Verfahren gemäß Anspruch 1, **dadurch gekennzeichnet, daß** die metallische Membran vor dem Einbringen zwischen die Elektroden mit einer Lösung getränkt wird.

3. Verfahren gemäß Anspruch 1, **dadurch gekennzeichnet, daß** das Dispergiermittel ein polares oder unpolares organisches Lösungsmittel oder Wasser ist.

4. Verfahren gemäß Anspruch 3, **dadurch gekennzeichnet, daß** das Lösungsmittel aus der Gruppe der Alkohole, Ester, Ether, organischen Säuren, gesättigten oder ungesättigten Kohlenwasserstoffe und Wasser ausgewählt ist oder eine Mischung hiervon ist, vorzugsweise, daß das Lösungsmittel aus der Gruppe bestehend aus Methanol, Ethanol, Propanol, Azeton und Wasser ausgewählt ist.

5. Verfahren gemäß Anspruch 1, **dadurch gekennzeichnet, daß** die Viskosität der Dispersion zwischen 1 und 1.000 mPa.s liegt, vorzugsweise zwischen 1 und 100 mPa.s liegt.

6. Verfahren gemäß Anspruch 1, **dadurch gekennzeichnet, daß** nacheinander verschiedene Dispersionen eingesetzt werden.

7. Verfahren gemäß Anspruch 1, **dadurch gekennzeichnet, daß** für die elektrophoretische Abscheidung elektrische Gleichspannungen von 5 V bis 100 V bzw. eine elektrische Feldstärke von 0,1 V/cm bis 20 V/cm angelegt werden.

8. Verfahren gemäß Anspruch 1, **dadurch gekennzeichnet, daß** die Abscheidedauer zwischen einer Sekunde und 30 Minuten beträgt.

9. Verfahren gemäß Anspruch 1, **dadurch gekennzeichnet, daß** die metallischen Formkörper nach der elektrophoretischen Abscheidung einer Sinterung unterzogen werden.

10. Verfahren gemäß Anspruch 9, **dadurch gekennzeichnet, daß** die Sintertemperatur zwischen 900°C und 1.700°C, vorzugsweise zwischen 1.000°C und 1.400°C, beträgt.

11. Verfahren gemäß einem der Ansprüche 1 bis 10, **dadurch gekennzeichnet, daß** der metallische Formkörper aus Nickel besteht.

12. Verfahren gemäß einem der Ansprüche 1 bis 11, **dadurch gekennzeichnet, daß** die keramischen Partikel Ceroxidpartikel (CeO₂) sind.

13. Verfahren gemäß Anspruch 12, **dadurch gekennzeichnet, daß** die keramischen Partikel Gadolinium dotierte Ceroxidpartikel (GDC) sind.

14. Verfahren gemäß einem der Ansprüche 1 bis 13, **dadurch gekennzeichnet, daß** die keramischen Partikel in bimodaler Verteilung vorliegen.

15. Metallischer Formkörper mit einer keramischen Schicht, **dadurch gekennzeichnet, daß** der metallische Formkörper eine gemäß einem der Ansprüche 1 bis 11 und 13 bis 14 hergestellte Nickel-GDC-Verbundmembran ist und nach der Trocknung eine graduelle Dichteänderung von 20 Vol.-% an der imprägnierten Oberfläche bis zu einer Tiefe von etwa 200 µm aufweist.

16. Verwendung eines metallischen Formkörpers gemäß Anspruch 15 insbesondere mit einer zusätzlich aufgebrachten keramischen Schicht, als Anode für Festoxid-Brennstoffzellen (SOFC).

## Claims

1. A process for the production of metallic shaped bodies with a ceramic layer according to the membrane method, in which a porous metallic membrane is arranged between the anode and the cathode in order to physically separate the change of the pH value in the region of the electrodes produced by H⁺ and OH⁻-ions from the location of the deposition, **characterized by the fact that** the porous metallic membrane is compacted by electrophoretic deposition of ceramic particles in the pores of the metallic membrane and the metallic membrane is arranged for electrophoretic deposition between two electrodes and the space between an electrode and the metallic membrane is filled with a dispersion containing the ceramic particles to be deposited in the pores and a dispersant.

2. Procedure according to Claim 1, **characterized by the fact that** the metallic membrane is saturated with a solution prior to be brought between the electrodes.

3. Procedure according to Claim 1, **characterized by the fact that** the dispersant is a polar or nonpolar organic solvent or water.

4. Procedure according to Claim 3, **characterized by the fact that** the solvent is selected from the group consisting of alcohols, Esters, Ethers, organic Acids, saturated or unsaturated Hydrocarbons and Water, or a mixture thereof, preferably that the solvent is selected from the group consisting of Methanol, Ethanol, Propanol, Acetone and Water.

5. Procedure according to Claim 1, **characterized by the fact that** the viscosity of the dispersion is situated between 1 and 1000 mPa.s, preferably between 1 and 100 mPa.s.

6. Procedure according to Claim 1, **characterized by the fact that** various different dispersions are used one after the other.

7. Claim according to Claim 1, **characterized by the fact that** for the electrophoretic separation electric constant voltage between 5 V and 100 V or an electric field of a strength of 0,1 V/cm up to 20 V/cm are applied.

8. Procedure according to Claim 1, **characterized by the fact that** the duration of the separation period is between one second and 30 minutes.

9. Procedure according to Claim 1, **characterized by the fact that** the metallic shaped bodies after the electrophoretic separation are subjected to a sintering process.

10. Procedure according to Claim 9, **characterized by the fact that** the sintering temperature is comprised between 900 °C and 1700 °C, preferably between 1000 °C and 1400 °C.

11. Procedure according to one of the Claims 1 to 10, **characterized by the fact that** the metallic shaped body consists of Nickel.

12. Procedure according to one of the Claims 1 to 11, **characterized by the fact that** the ceramic particles are Ceriumoxide (CeO₂) particles.

13. Procedure according to Claim 12, **characterized by the fact that** the ceramic particles are Gadolinium doped Ceria (GDC) particles.

14. Procedure according to one of the Claims 1 to 13, **characterized by the fact that** the ceramic particles exist in bimodal distribution.

15. Metallic shaped body with a ceramic layer, **characterized by the fact** that the metallic shaped body is a Nickel-GDC-Compound Membrane made according to one of the Claims 1 to 11 and 13 to 14, which after drying shows a gradual change of density of 20 vol.-% in the impregnated surfaces down to a depth of about 200 µm.

16. Use of a metallic shaped body according to Claim 15 especially with an additional applied ceramic layer, as an anode for Solid Oxide Fuel Cells (SOFC).

## Revendications

1. Procédé de fabrication de corps formés métalliques avec une couche de céramique suivant le procédé à membrane, dans lequel une membrane métallique poreuse est placée entre l'anode et la cathode afin de séparer dans l'espace le changement de la valeur pH induit par les ions H⁺ et OH⁻ dans la zone des électrodes et le site de dépôt, **caractérisé par le fait que** la membrane métallique poreuse est recomprimé par un dépôt électrophorétique des particules de céramique dans les pores de la membrane métallique et que le diaphragme métallique pour le dépôt électrophorétique est disposé entre les deux électrodes et que l'espace entre une électrode et la membrane métallique est rempli d'une dispersion qui contient les particules de céramique à déposer dans les pores ainsi qu'un agent dispersant.

2. Procédé suivant Revendication 1, **caractérisé par le fait que** la membrane métallique est trempée d'une solution avant d'être introduite entre les électrodes.

3. Procédé suivant Revendication 1, **caractérisé par le fait que** l'agent dispersant consiste d'un solvant organique polaire ou non-polaire ou alors de l'eau.

4. Procédé suivant la Revendication 3, **caractérisé par le fait que** le solvant est choisi dans le groupe des alcools, des esters, des éthers, des acides organiques, saturés ou non saturés et de l'eau, ou qu'il s'agit d'un mélange de ceux-ci, de préférence que le solvant est choisi dans le groupe constitué par le méthanol, l'éthanol, le propanol, l'acétone et l'eau.

5. Procédé suivant Revendication 1, **caractérisé par le fait que** la viscosité de la dispersion est comprise entre 1 et 1.000 mPa.s, de préférence entre 1 et 100 mPa.s.

6. Procédé suivant Revendication 1, **caractérisé par le fait que** des dispersions différentes sont utilisées l'une après l'autre.

7. Procédé suivant Revendication 1, **caractérisé par le fait que** pour le dépôt électrophorétique des courants constants entre 5 V et 100 V ou alors un champ électrique de 0,1 V/cm jusqu'à 20 V/cm sont mis en places.

8. Procédé suivant Revendication 1, **caractérisé par le fait que** la durée du dépôt est comprise entre une seconde et 30 minutes.

9. Procédé suivant Revendication 1, **caractérisé par le fait que** les corps métalliques formés sont après le dépôt électrophorétique assujettis à un frittage.

10. Procédé suivant Revendication 9, **caractérisé par le fait que** la température de frittage est comprise entre 900 °C et 1700 °C, de préférence entre 1000 °C et 1400 °C.

11. Procédé suivant l'une des Revendications 1 à 10, **caractérisé par le fait que** le corps formé métallique consiste de Nickel.

12. Procédé suivant l'une des Revendications 1 à 11, **caractérisé par le fait que** les particules céramiques sont des particules de Céroxide (CeO₂).

13. Procédé suivant Revendication 12, **caractérisé par le fait que** les particules céramiques sont des particules de Céroxide dotés de Gadolinium (GDC).

14. Procédé suivant l'une des Revendications 1 à 13, **caractérisé par le fait que** les particules céramiques sont distribués de manière bimodale.

15. Corps métallique formé avec une couche céramique, **caractérisé par le fait que** le corps métallique formé est une membrane composite Nickel-GDC fabriquée d'après l'une quelconque des Revendications 1 à 11 et 13 à 14 et qui après séchage montre une modification progressive de la densité de 20 vol.-% sur la surface imprégnée allant jusqu'à une profondeur d'environ 200 µm.

16. Utilisation d'un corps formé métallique d'après Revendication 15 et particulièrement avec une couche supplémentaire céramique, destiné comme anode pour les Piles à combustible faites d'Oxide solide (SOFC).
